# EUROPEAN PATENT SPECIFICATION

(11) **EP 0 381 353 B1**
(45) Date of publication and mention of the grant of the patent: **07.12.1994**
(21) Application number: 90300677.3
(22) Date of filing: 23.01.1990
(51) Int. Cl.: C07C 13/48, C07C 2/72, C07C 2/66

(54) **Process for preparing polyalkyl tetrahydronaphthalenes**
Verfahren zur Herstellung von Polyalkyltetrahydronaphthalenen
Procédé pour la préparation de polyalkyl-tétrahydro-naphtalènes

(30) Priority: 27.01.1989 US 303364; 27.01.1989 US 303355
(43) Date of publication of application: 08.08.1990
(73) Proprietor: Union Camp Corporation, Wayne New Jersey 07470 - 2066 (US)
(72) Inventor: Frank, Walter C., Holland PA 18966 (US)
(74) Representative: Jones, Helen Marjorie Meredith

(56) References cited:
- US-A- 4 551 573
- US-A- 4 877 915
- CHEMICAL ABSTRACTS, vol. 97, no. 3, August 1982, page 545, abstract no. 38653f,Columbus, Ohio, US; & JP-A-82 40 420
- CHEMICAL ABSTRACTS, vol. 95, no. 17, October 1981, page 630, abstract no. 150266s, Columbus, Ohio, US; & JP-A-81 39 026

## Description

The present invention relates to an improved process for the production of polyalkyl tetrahydronaphthalenes, particularly 1,1,3,4,4,6-hexamethyl-1,2,3,4-tetrahydronaphthalene, the latter compound referred to herein as "HMT".

HMT and other alkyl-substituted tetrahydronaphthalenes are of significant importance to the perfumery as well as other industries. By conventional acylation processes, HMT, for example, can be converted to 7-acetyl-1,1,3,4,4,6-hexamethyl-1,2,3,4-tetrahydronaphthalene, a well known musk perfume. Because of their clean musk fragrance and their ability to retain that fragrance over long periods of time, these HMT derivatives are of great commercial value as synthetic musk perfume substitutes for the expensive, natural musk perfumes of the macrocyclic ketone series. Consequently, various synthetic methods have been proposed for the production of HMT, as well as other intermediates of HMT useful in the perfumery or other industries.

For example, Cobb, U.S. Patent No. 4,551,573 discloses a process for the alkylation of aromatic compounds with olefinic compounds in the presence of a catalyst consisting essentially of aluminum halide and elemental iodine. Examples of aromatic compounds described as suitable for use in the process include para-cymene, and olefinic compounds discussed include 2,3-dimethyl-2-butene, isobutylene and neohexene (3,3-dimethyl-1-butene). A mixture of olefinic compounds can also be employed, in which case it is noted that one of the olefins may function as a sacrificial agent. The products of the alkylation reaction described include indanes and HMT-type compounds.

Japanese Patent Publication SHO 57-40420 discusses a method of making HMT characterized by reacting para-cymene and neohexene in the presence of anhydrous aluminum halide as catalyst. Suitable anhydrous aluminum halides are said to include aluminum chloride. The reaction is generally carried in a solvent, however, it is noted that it is possible to conduct the reaction without any additional solvent using excess para-cymene. Examples of suitable solvents are methylene chloride, ethylene chloride, chloroform and other inactive fatty hydrocarbon halides. Other solvents such as aromatic hydrocarbon halides, fatty hydrocarbons, aromatic hydrocarbons, etc., can be used, but it is noted that the use of such solvents generally lowers the yield of the desired end product.

Wood, U.S. Patent No. 3,246,044, discloses a process for preparing HMT which includes reacting an alpha,para-dimethylstyrene derivative such as dimethyl-para-tolyl-carbinyl halide, and neohexene in the presence of a catalyst such as aluminum chloride, aluminum bromide and ferric chloride, or other Friedel-Crafts catalysts, at low temperatures. Suitable solvents are listed as ethylene dichloride or carbon tetrachloride, or other inert chlorinated hydrocarbon solvents. It is noted that other solvents such as nitrobenzene and nitromethane, may be used, but the yield of desired product is indicated as generally being lower when such solvents are employed.

Wood et al., U.S. Patent No. 3,856,875, discusses a process for the preparation of HMT wherein an equivalent or excess amount of para-cymene is reacted with a substantially equal molar solution of neohexene and a tertiary alkyl halide in the presence of an effective amount of an anhydrous aluminum halide catalyst suspended in a reaction-compatible solvent. Although any tertiary alkyl halide can be employed in the disclosed process, tertiary butyl chloride, tertiary amyl chloride and 2,5-dichloro-2,5-dimethylhexane are noted as preferred. The process is described as having a solvent dependency, with the satisfactory solvents being ethylene dichloride, chloroform, methylene dichloride, 1,1,2,2-tetrachloroethane, 1,2-dichloroethylene, 1,2,3-trichloropropane, 1,1,2-trichloroethane, monochlorobenzene, fluorobenzene, ortho-dichlorobenzene, and para-xylene. Numerous solvents were deemed unsatisfactory for use in the disclosed process, such solvents including nitromethane, benzene, nitrobenzene, para-cymene, n-hexane, 1,2,2-trichloroethylene, carbon tetrachloride, 1,1,1-trichloromethane, carbon disulfide, 1,1,2,2,2-pentachloroethane, 1,2-dichloropropane, 1,1-dichloroethylene, and 1,1-dichloroethane. These unsatisfactory solvents are said to yield substantially poorer results.

Sato et al., U.S. Patent No. 4,284,818, describes a process for producing HMT comprising reacting para-cymene with a 2,3-dimethyl butene using a catalytic amount of anhydrous aluminum halide in the presence of a secondary alkyl halide, tertiary alkyl halide, propargyl halide or allyl halide. It is noted that both the 2,3-dimethyl-1-butene and 2,3-dimethyl-2-butene can be employed as the 2,3-dimethyl butene reagent, however, 2,3-dimethyl-1-butene was said to yield better results. The reaction is generally carried out using a solvent, such solvents including aliphatic hydrocarbons, halogenated aromatic hydrocarbons, and halogenated aliphatic hydrocarbons.

Kahn, U.S. Patent No. 3,379,785, relates to a process for preparing polyalkyl tetrahydronaphthalenes, and more specifically, a process for preparing HMT. The process involves the reaction of a substituted styrene and a 2,3-dimethylbutene, said reaction being carried out at elevated temperatures and in the presence of a cation exchange resin. The 2,3-dimethylbutene reactant employed is disclosed as comprising either 2,3-dimethyl-1-butene, 2,3-dimethyl-2-butene, or mixtures thereof. The preferably employed solvent comprises an aromatic hydrocarbon, such as, for example, benzene, toluene, ethylbenzene, or a xylene.

Suzukamo et al., U.S. Patent No. 4,767,882, discloses a process for preparing a tetrahydronaphthalene derivative in an optically active state which comprises reacting a benzene compound and a pyrocine compound in the presence of a Lewis acid, or, alternatively, reacting the benzene with the pyrocine compound in the presence of an acid catalyst followed by treatment of the resultant product with the Lewis acid.

These prior art processes suffer from various drawbacks, including low conversion of reactants, poor selectivity to the desired products, sluggish reaction rates, unacceptably low temperature requirements, unsafe solvent systems, or oxygen sensitivity. New and/or better processes are needed. The present invention is directed to this important end.

### SUMMARY OF THE INVENTION

The present invention provides a process for the production of polyalkyl tetrahydronaphthalenes wherein a cyclialkylation reaction between an olefinic compound of the general Formula 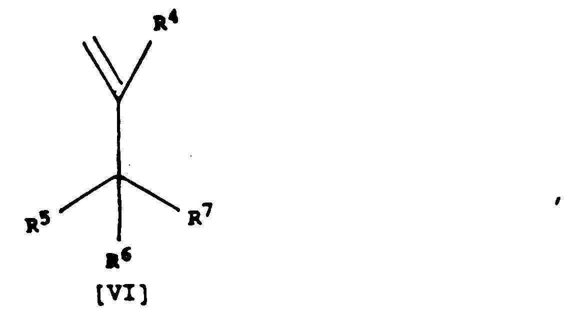
and a substituted benzene compound is carried out in the presence of a hydride abstracting reagent, a Lewis acid, and, optionally, a phase transfer agent. In some embodiments, the subject process is specifically carried out in the absence of elemental iodine. The subject process, which in some embodiments may preferably be practiced in an unhalogentated hydrocarbon solvent, produces the desired compounds in a surprisingly high yield, with a surprisingly high selectivity to the desired product, and at a relatively high rate of reaction, using better, more convenient or less expensive process methodology than many processes known heretofore.

In the present invention there is provided a new process for producing a polyalkyl tetrahydronaphthalene compound comprising contacting a partially substituted benzene compound, wherein said benzene compound is substituted with two or more substituents that do not substantially interfere with a Friedel-Crafts-type alkylation reaction said substituents including at least one secondary alkyl group having only one alpha-hydrogen, and wherein said benzene compound is unsubstituted in at least one position adjacent to said secondary alkyl group, with an olefinic compound of the Formula 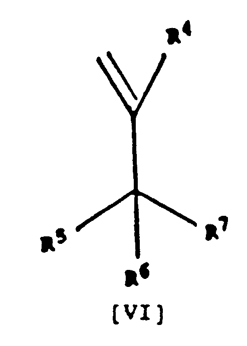
wherein R⁴, R⁵, R⁶ and R⁷ independently are substituents that do not substantially interfere with a Friedel-Crafts-type alkylation reaction provided that no more than one of R⁵, R⁶ and R⁷ are H, in the presence of
a further olefin reagent provided that said further olefin reagent has greater electron releasing properties than said olefinic compound of the formula VI, and
a Lewis acid,
characterised in that the further olefin reagent has the formula VII 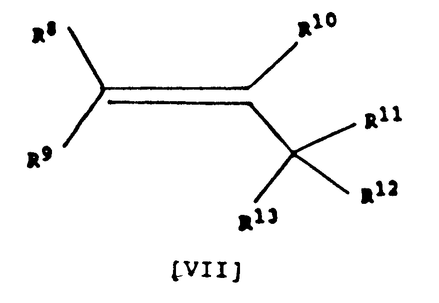
wherein
R⁸, R⁹, R¹⁰, R¹¹, R¹² and R¹³, independently, are substitutents that do not substantially interefere with a Freidel-Crafts-type alkylation reaction, provided that
(i) no more than one of R⁸, R⁹ and R¹⁰ are H, and
(ii) no more than one of R¹¹, R¹² and R¹³ are H.

In one preferred embodiment of the invention the process is carried out in the substantial absence of elemental iodine.

The process may, if desired, be carried out in the presence of a phase transfer agent, with or without the substantial absence of elemental iodine.

Using the foregoing processes, one is able to produce a variety of alkyl-substituted tetrahydronaphthalene compounds for use as chemical intermediates and/or chemical products, particularly intermediates such as HMT, which is a compound of extreme importance to the fragrance industry.

### DETAILED DESCRIPTION OF THE INVENTION

As noted above, the present invention pertains to a novel and particularly useful process for the production of polyalkyl tetrahydronaphthalenes including, but not limited to, those of Formulas I, II or III:

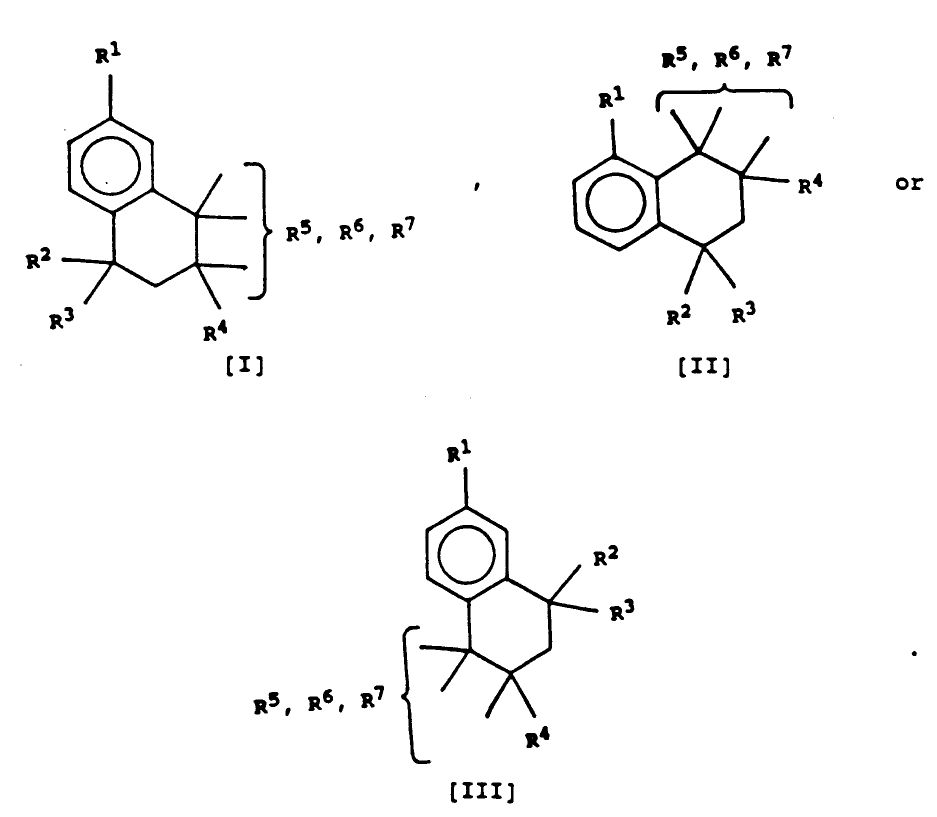

In the above Formulas, R¹, R², R³, R⁴, R⁵, R⁶ and R⁷ are defined, independently, as substituents that do not substantially interfere with a Friedel-Crafts-type alkylation reaction, provided that R¹, R² and R³ are each other than H, and no more than one of R⁵, R⁶ and R⁷ are H. The bracket notation as employed in Formulas I, II and III signifies that each of substituents R⁵, R⁶ and R⁷ can be present at any one of the attachment positions contained within the brackets, but not at more than one of these positions. In other words, the three attachment positions within the brackets are satisfied with an R substituent, one attachment position being satisfied with an R⁵ substituent, another with an R⁶ substituent, and a third with an R⁷ substituent. Suitable R¹, R², R³, R⁴, R⁵, R⁶ and R⁷ substituents will be readily apparent to those skilled in the art of Friedel-Crafts-type alkylation reactions. Such alkylation reactions and non-interfering substituents are discussed, for example, in George A. Olah, Friedel-Crafts and Related Reactions, Vols. 1 and 2 (Interscience Publishers, John Wiley and Sons, 1964) (hereinafter referred to as "Friedel-Crafts Reactions"), as well as in other journal and textbook references. The disclosures of Friedel-Crafts Reactions are incorporated herein by reference. Examples of suitable substituents include those wherein R¹, R² and R³, independently, are a C₁-C₃₀ straight chain, branched or cyclical alkyl, R⁴ is H, or a C₁-C₃₀ straight chain, branched or cyclical alkyl, and R⁵, R⁶ and R⁷, independently, are H or a C₁-C₃₀ straight chain, branched or cyclical alkyl, provided that no more than one of R⁵, R⁶ and R⁷ are H. The alkyl is preferably a C₁-C₂₀, more preferably a C₁-C₁₀, and most preferably a C₁-C₅, alkyl. Preferably, the alkyl is a straight chain or branched alkyl. In a generally preferred embodiment, R¹, R² and R³, independently, are a C₁-C₅ straight chain or branched alkyl, R⁴ is H or a C₁-C₅ straight chain or branched alkyl, and R⁵, R⁶, and R⁷ are H or a C₁-C₅ straight chain or branched alkyl, provided that no more than one of R⁵, R⁶ and R⁷ are H.

In a most preferred embodiment, the polyalkyl tetrahydronaphthalenes are of the Formula I. The Formula I compounds are preferably:
o 1,1,3,4,4,6-hexamethyl-1,2,3,4-tetrahydronaphthalene (that is, HMT, a compound of Formula I wherein R¹, R², R³, R⁵, R⁶ and R⁷ are each methyl, and R⁴ is H);
o 6-ethyl-1,1,3,4,4-pentamethyl-1,2,3,4-tetrahydronaphthalene (that is, a compound of Formula I wherein R¹ is ethyl, and R², R³, R⁵, R⁶ and R⁷ are each methyl, and R⁴ is H);
o 6-tertiary-butyl-1,1,3,4,4-pentamethyl-1,2,3,4-tetrahydronaphthalene (that is, a compound of Formula I wherein R¹ is tertiary butyl, and R², R³, R⁵, R⁶ and R⁷ are each methyl, and R⁴ is H); and
o 6-n-propyl-1,1,3,4,4-pentamethyl-1,2,3,4-tetra-hydronaphthalene (that is, a compound of Formula I wherein R¹ is n-propyl, and R², R³, R⁵, R⁶ and R⁷ are each methyl, and R⁴ is H).

The compounds of Formulas I, II and III are produced by contacting a partially substituted benzene compound, wherein said benzene compound is substituted with two or more substituents that do not substantially interfere with a Friedel-Crafts-type alkylation reaction said substituents including at least one secondary alkyl group having only one alpha-hydrogen, and wherein said benzene compound is unsubstituted in at least one position adjacent to said secondary alkyl group, such substituted benzene compounds including, but not limited to, those compounds of the Formulas IV or V 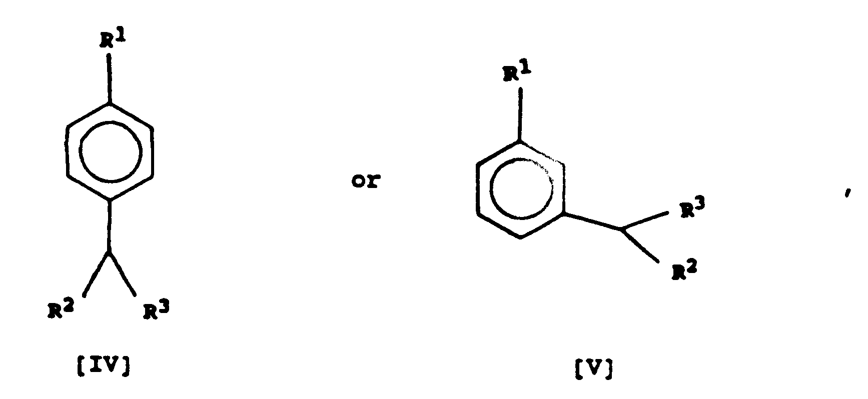
with an olefinic compound of the Formula VI

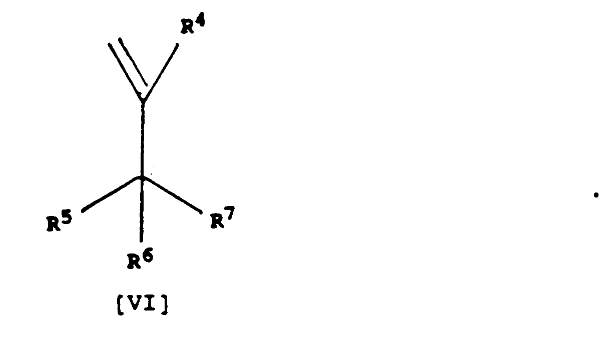

Contacting a benzene compound of Formula IV with an olefinic compound of Formula VI will yield the tetrahydronaphthalene compounds of Formula I. Alternatively, contacting a benzene compound of Formula V with an olefinic compound of Formula VI will yield the tetrahydronaphthalene compounds of Formulas II and III. The Formula I, II or III compounds may isomerize under the reaction conditions to also form compounds of one or more of the other Formula I, II or III compounds.

In the above Formulas IV, V and VI, R¹, R², R³, R⁴, R⁵, R⁶ and R⁷ are defined, independently, as previosly described, that is, as substituents that do not substantially interfere with a Friedel-Crafts-type alkylation reaction, provided that R¹, R² and R³ are each other than H, and no more than one of R⁵, R⁶ and R⁷ are H. Suitable substituents are discussed in various journal and textbook references, such as Friedel-Crafts Reactions. Suitable substituents include those wherein R¹, R² and R³, independently, are a C₁-C₃₀ straight chain, branched or cyclical alkyl, R⁴ is H, or a C₁-C₃₀ straight chain, branched or cyclical alkyl, and R⁵, R⁶ and R⁷, independently, are H, or a C₁-C₃₀ straight chain, branched or cyclical alkyl, provided that no more than one of R⁵, R⁶ and R⁷ are H. The alkyl is preferably a C₁-C₃₀, more preferably a C₁-C₁₀, and most preferably a C₁-C₅, alkyl. Preferably the alkyl is a straight chain or branched alkyl.

With respect to the benzene compounds of Formulas IV and V, a generally preferred embodiment includes those compounds wherein R¹, R² and R³, independently, are a C₁-C₅ straight chain or branched alkyl. In a most preferred embodiment, the substituted benzene compounds are of Formula IV. The Formula IV compounds are preferably isopropyl toluene (that is, para-cymene, a compound of Formula IV wherein R¹, R² and R³ are each methyl), 1-ethyl-4-isopropylbenzene (that is, a compound of Formula IV wherein R¹ is ethyl, and R² and R³ are each methyl), 1-n-propyl-4-isopropylbenzene (that is, a compound of Formula IV wherein R¹ is n-propyl, and R² and R³ are each methyl), and 1-tertiary-butyl-4-isopropylbenzene (that is, a compound of Formula IV wherein R¹ is tertiary-butyl, and R² and R³ are each methyl).

In a generally preferred embodiment, the olefinic compounds of Formula VI include those compounds wherein R⁴ is H or a C₁-C₅ straight chain or branched alkyl, and R⁵, R⁶ and R⁷ independently, are H or a C₁-C₅ straight chain or branched alkyl, provided that no more than one of R⁵, R⁶ and R⁷ are H. A more preferable embodiment is wherein R⁴ is H or methyl. Of the Formula VI compounds, 3,3-dimethyl-1-butene (that is, neohexene, a compound of Formula VI wherein R⁴ is H, and R⁵, R⁶ and R⁷ are each methyl) and 2,3-dimethyl-1-butene (a compound of Formula VI wherein R⁴, R⁵ and R⁶ are each methyl, and R⁷ is H) are most preferred. As those skilled in the art would recognize, certain internal olefinic isomers of the terminal olefins of Formula VI can be employed in lieu of the Formula VI compounds. Such compounds are capable of rearranging by isomerization under the process conditions of the invention to form the terminal olefinic Formula VI compounds believed to be required for tetrahydronaphthalene formation. A preferable internal olefinic isomer is 2,3-dimethyl-2-butene.

In accordance with the present invention, the compounds of Formulas IV or V are contacted with compounds of Formula VI in the presence of a reagent of the Formula 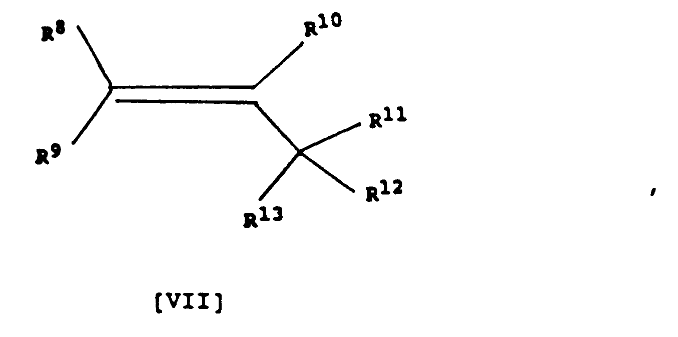
provided that said reagent has greater electron releasing properties than said olefinic compound, and further in the presence of a Lewis acid, and, optionally, a phase transfer agent.

In the above Formula VII, R⁸, R⁹, R¹⁰, R¹¹, R¹² and R¹³ are defined, as previously described, that is, as substituents that do not substantially interfere with a Friedel-Crafts-type alkylation reaction, provided that no more than one of R⁸, R⁹ and R¹⁰ are H, and no more than one of R¹¹, R¹² and R¹³ are H. Suitable substituents include those wherein R⁸, R⁹, R¹⁰, R¹¹, R¹² and R¹³ are, independently, H or a C₁-C₃₀ straight chain, branched or cyclical alkyl. The alkyl is preferably a C₁-C₂₀, more preferably a C₁-C₁₀ and most preferably a C₁-C₅ alkyl. Preferably the alkyl is a straight chain or branched alkyl. In a most preferred embodiment, the Formula VII compound is 2,4,4-trimethyl-2-pentene (that is, diisobutylene-2, a compound of Formula VII wherein R⁸, R⁹, R¹¹, R¹² and R¹³ are methyl and R¹⁰ is H). The particular reagents defined in Formula VII have been found to be surprisingly effective hydride abstractors. These compounds are capable of preferentially carrying out the hydride abstraction function, rather than participating in the alkylation step. This results in a process which has a smaller amount of side reactions occurring, and thus a higher selectivity to and yield of the desired end product.

As noted above, the Formula VII compounds employed in a process of the invention must have greater electron releasing properties than the olefinic compounds VI also utilized in that process. The comparative electron releasing properties of the Formula VI and VII compounds will be readily apparent to those skilled in the art. As will be recognized, for example, any of the Formula VII compounds wherein the R⁸ through R¹³ substituents are selected from H or alkyl, will have greater electron releasing properties than any of the Formula VI compounds wherein the R⁴ through R⁷ substituents are also selected from H or alkyl. Accordingly, it is expected that the Formula VII compounds will function in the present process as the primary hydride abstracting agents, relieving the olefinic compounds VI of this task and enabling the Formula VI olefins to instead function as alkylating agents. In addition, by utilizing the Formula VII compounds in accordance with the present process, one will be employing in a non-productive reduction (hydride abstraction) step a more readily available, less expensive reagent VII, in lieu of the less readily available, more expensive alkyl halide compounds consumed in accordance with some prior art procedures. As a result, it is possible to avoid by-product formation of hydrogen halides and accumulation of such compounds in the product stream, an undesirable result associated with some art processes. Moreover, the potential for corrosion problems within the reaction system concomitant with the formation of the hydrogen halides may be lessened, and the need for complex procedures for the separation of the desired HMT-type products from the hydrogen halide by-products may be minimized.

Any Lewis acid, that is, any non-protonic compound capable of accepting an electron pair, is suitable for use in the present process. Exemplary Lewis acids include metal halides such as aluminum halides (including aluminum chloride, aluminum bromide, aluminum iodide, monofluorodichloroaluminum, monobromodichloroaluminum and monoiododichloroaluminum), alkyl metal halides and alkyl metals. Alkyl metals and alkyl metal halides suitable for use as Lewis acids in the present process are disclosed, for example, in Kennedy, Joseph P., Carbocationic Polymerization, p. 221 (Wiley-Interscience Publishers (1982)), the disclosures of which are incorporated herein by reference. In the process of the present invention, aluminum halides are preferred. Of the aluminum halides, aluminum chloride and aluminum bromide, particularly aluminum chloride, are most preferred.

In a preferable embodiment, the reaction is carried out in the presence of a phase transfer agent. Suitable phase transfer agents include onium salts such as ammonium, phosphonium and sulfonium salts. Other phase transfer agents suitable for use in the present process will be readily apparent to those skilled in the art, once having been made aware of the present disclosure.

Examples of ammonium phase transfer agents include quaternary ammonium halides such as methyltrioctylammonium chloride, methyltrinonylammonium chloride, methyltridecylammonium chloride, hexadecyltrihexylammonium bromide, ethyltrioctylammonium bromide, didodecyldimethylammonium chloride, tetraheptylammonium iodide, dioctadecyldimethylammonium chloride, tridecylbenzylammonium chloride, ditricosylmethylammonium chloride, and homologues thereof having chlorine, fluorine, bromine or iodine atoms substituted for the enumerated halide atom.

Exemplary phosphonium phase transfer agents include quaternary phosphonium halides such as tributyldecylphosphonium iodide, triphenyldecylphosphonium iodide, tributylhexadecylphosphonium iodide, and homologues thereof having chlorine, fluorine or bromine atoms substituted for the iodine atom.

Representative sulfonium phase transfer agents include ternary sulfonium halides such as lauryldimethylsulfonium iodide, lauryldiethylsulfonium iodide and tri(n-butyl)sulfonium iodide, and homologues thereof having chlorine, fluorine or bromine atoms substituted for the iodine atom.

These and other suitable phase transfer agents are described, for example, in Napier, U.S. Patent No. 3,992,432 and in Kondo et al., Synthesis, pp. 403-404 (May 1988), the disclosures of which are incorporated herein by reference.

Preferable phase transfer agents are ammonium or sulfonium salts, particularly quaternary ammonium or ternary sulfonium halides. Most preferred are quaternary ammonium halides, particularly methyltrioctylammonium chloride (referred to herein as "MTOAc"), and a mixture of methyltrioctylammonium chloride and methyltridecylammonium chloride. The latter mixture is marketed under the tradename Adogen-464™, by Sherex Co., located in Dublin, Ohio.

In general, the molar proportions of the reagents employed in the present process can be varied over a relatively wide range. However, where phase transfer agents are employed in the process, it is important, for the best results, to maintain a ratio of less than one mole of phase transfer agent per mole of Lewis acid. Preferably, the molar ratio is about 0.8 to 1.0, more preferably 0.5 to 1.0, phase transfer agent to Lewis acid. It should be noted that some phase transfer agents sold commercially are sold in an impure form. Such impurities generally comprise water or an alcohol species. As those skilled in the art will recognize, water and alcohol, as well as other impurities which will be readily apparent to those skilled in the art, will react adversely with the Lewis acid, thereby lowering the amount of active Lewis acid available for the process of the present invention. Accordingly, where the phase transfer agent added contains such impurities, the amount of Lewis acid should thus preferably be increased to account for these impurities. In such a situation the ratio of transfer agent to Lewis acid might be about 0.3 to 1.0. Such impure agent-containing mixtures are referred to herein as mixtures in an "impure form".

It is preferable to use a mixture of olefinic compound VI and hydride abstracting reagent VII wherein these components are present in a molar range of about 1.0 to about 5.0 moles of olefin VI per mole of reagent VII. More preferably, the olefin VI and reagent VII are present in nearly equimolar amounts, that is, about 1.0 mole of olefin VI per mole of reagent VII.

Preferably, the substituted benzene compound is present in a range of about 0.5 to about 10 moles per mole of olefin VI. More preferably, the substituted benzene compound is present in a range of about 0.5 to about 5.0 per mole of olefin VI.

In a most preferred embodiment, each of the benzene compound, olefin VI, and the hydride abstracting reagent VII, are present nearly in equimolar amounts, that is, about 1.0 mole of benzene compound, to about 1.0 mole of olefin VI, to about 1.0 mole of hydride abstracting reagent VII.

The amount of Lewis acid utilized is preferably in the range of about 2% to about 10% by weight of the Lewis acid based on the combined weight of the substituted benzene, olefin VI, and hydride abstracting reagent VII.

As noted above, in certain embodiments, the present process is conducted in the substantial absence of elemental iodine (I₂). By "substantial absence", it is meant that only a deminimus amount of iodine (such as, for example, less than 1% by weight of I₂ based on the weight of the Lewis acid), if any, is present in the reaction medium. Preferably, in the embodiments which require a substantial absence of iodine, the reaction medium is devoid of any elemental iodine.

The reaction is generally carried out using a solvent, although, if desired, substituted benzene, one of the starting materials, may be employed in large excess in lieu of an additional solvent. A number of different solvents may be utilized in the present invention, including halogenated and unhalogenated aliphatic, alicyclic and aromatic hydrocarbon solvents.

Where the process is run in the absence of a phase transfer agent, and the olefinic compound of Formula VI is one wherein R⁵, R⁶ and R⁷ each are other than H, such halogenated aliphatic, halogenated alicyclic and halogenated aromatic hydrocarbon solvents ore preferred, for reasons of increased yield. Representative of the halogenated solvents are the aliphatic solvents methylene chloride, chloroform, carbon tetrachloride, ethylene chloride, ethylidene chloride, 1,1,1-trichloroethane, 1,1,2-trichloroethane, 1,1,2,2-tetrachloroethane, 1,2-dichloroethylene, trichloroethylene, tetrachloroethylene, 1,2,3-trichloropropane, amyl chloride, and ethylene bromide, and the aromatic solvents monochlorobenzene, ortho-dichlorobenzene, bromobenzene and fluorobenzene.

Where a phase transfer agent is employed in connection with the subject process or the olefinic compound of Formula VI is one wherein one of R⁵, R⁶ and R⁷ is H, the unhalogenated aliphatic, unhalogenated alicyclic and unhalogenated aromatic hydrocarbon solvents are preferred, for reasons of increased yield, safety and/or process engineering. Exemplary of the unhalogenated solvents are the aliphatic solvents n-hexane, n-heptane and n-octane, the alicyclic solvent cyclohexane, and the aromatic solvents benzene, toluene, ethylbenzene and xylene. Particularly preferred for reasons of yield, safety and/or process engineering are the unhalogenated aliphatic and unhalogenated alicyclic hydrocarbons. Other suitable halogenated and unhalogenated solvents are described, for example, in U.S. Patent Nos. 4,284,818, 3,856,875 and 3,379,785, the disclosures of which are incorporated herein by reference.

The alkylation reaction of the invention can be carried out in any suitable vessel which provides efficient contacting between the Lewis acid and the other reactants. For simplicity, a stirred batch reactor can be employed. Although stirring is recommended to provide efficient contact between reactants, it has been found that with the addition of the phase transfer agent pursuant to one embodiment of the present invention, the Lewis acid is able to solubilize rather quickly, thereby obviating the need for the stringent stirring requirements of many of the art processes utilized to produce HMT. The reaction vessel used should be resistant to the possibly corrosive nature of the catalyst. Glass-lined vessels are suitable for this purpose, as well as other vessel materials well-known in the art.

The reagents of the present process may be added in any order, although where the process is carried out with a phase transfer agent, the preferred mode is to add the solvent, the Lewis acid and the phase transfer agent first, allow sufficient time for the Lewis acid to become substantially dissolved in the solvent, and then add the remaining reagents. Generally, 15 to 30 minutes are needed for the Lewis acid to become substantially dissolved in the solvent.

Ideally, the reaction is carried out at temperatures ranging from about -30°C to about 50°C, preferably at temperatures ranging from about -10°C to about 40°C, and most preferably at temperatures ranging from about 0°C to about 30°C.

The pressure at which the reaction is carried out is not critical. If the reaction is carried out in a sealed vessel, autogenous pressure is acceptable, although higher or lower pressures, if desired, may be employed. The reaction can also be carried out at atmospheric pressure in an open reaction vessel, in which case the vessel is preferably equipped with a moisture trap to prevent significant exposure of Lewis acid to moisture. The reaction can take place in an oxygen atmosphere, or an inert atmosphere as in the presence of a gas such as nitrogen, argon and the like, the type of atmosphere also not being critical.

Reaction time is generally rather short and is often dictated by the kind of equipment employed. Sufficient time must be provided, however, for thorough contacting of the substituted benzene compound, the olefinic compound, the Lewis acid and the phase transfer agent. Generally the reaction proceeds to completion in about 1 to about 7 hours.

Product can be recovered by first quenching the reaction mixture in cold water or on crushed ice, preferably on ice, and then processing the mixture in the usual manner for Friedel-Crafts reactions to extract the desired alkyl-substituted tetrahydronaphthalene compounds. Suitable extraction protocol is described, for example, in Friedel-Crafts Reactions. Typically, following quenching and the resultant phase separation, the organic layer is washed an additional time with water to aid in removal of the Lewis acid. One or more additional washings can be carried out with dilute alkali solution to further aid Lewis acid removal. Pure product can then be recovered by subjecting the washed reaction mixture to reduced pressure fractional distillation.

The polyalkyl tetrahydronaphthalenes prepared in accordance with the processes of the invention, as hereinbefore indicated, may, for example, be acylated to obtain acylated polyalkyl tetrahydronaphthalenes having very fine, musk-like fragrances, a characteristic which renders them highly valuable for use in the perfumery industry. Such products, acylated or otherwise, may alternatively or additionally have utility in the pharmaceutical and/or agrochemical industries, either as intermediates or as end products, as generally discussed in French Patent Publication No. 2601670, and Cobb, U.S. Patent No. 4,551,573. The acylation process may be carried out in using conventional methods, such as by reacting the polyalkyl tetrahydronaphthalene with an acyl halide or acid anhydride in the presence of an acid-acting catalyst. Suitable acylation methods are well known in the art and are disclosed, for example, in Sato et al., U.S. Patent No. 4,284,818. Examples of acylated polyalkyl tetrahydronaphthalenes include 7-acetyl-1,1,3,4,4,6-hexamethyl-1,2,3,4-tetrahydronaphthalene, 7-acetyl-1,1,3,4,4-pentamethyl-6-ethyl-1,2,3,4-tetrahydronaphthalene, 7-acetyl-1,1,3,4,4-pentamethyl-6-n-propyl-1,2,3,4-tetrahydronaphthalene, and 7-acetyl-1,1,3,4,4-pentamethyl-6-tertiary-butyl-1,2,3,4-tetrahydronaphthalene.

The present invention is further described in the following Examples.

In each Example, the reaction flasks were equipped with a condenser, mechanical stirrer, addition funnel and thermocouple/temperature controller connected to an automatic laboratory jack. The flasks were cooled, when necessary, with a dry ice/isopropanol bath. The flask contents were continuously stirred throughout the reaction.

Results were analyzed on both polar and non-polar gas chromatography columns. All gas chromatography analyses were carried out on capillary columns using a weight percent internal standard method of analysis. Structure identifications were assigned based on GCMS fragmentation patterns compared to standards.

Examples 1-15 are examples of processes of the present invention.

### Examples

### Example 1

A 100 ml, four-necked, round bottom flask was charged with cyclohexane (7.17 g) and anhydrous aluminum chloride (1.004 g), and cooled to about 16°C. A 60 ml addition funnel was charged with para-cymene (39.87 g), diisobutylene-2 (8.34 g), and neohexene (6.25 g) and connected to the flask. The funnel mixture was added to the flask over a period of about one hour and the flask mixture stirred 30 minutes following addition, while maintaining the temperature at about 16°C. The reaction was then quenched with deionized water (15 ml), and the organic phase separated and washed with, in order, 5% HCl, 10% Na₂CO₃ and 50% (that is, half-saturated) brine. The aqueous layers were individually extracted with ethyl ether, and the ether layers combined with the organic phase. The organics were then dried over K₂CO₃, filtered, and evaporated to yield a crude product (41.13 g) containing 28.16 weight % HMT (73.30% molar yield of HMT based on the amount of neohexene charged).

### Example 2

A 100 ml, four-necked, round bottom flask was charged with Adogen-464™ (1.063 g), cyclohexane (7.15 g) and anhydrous aluminum chloride (1.007 g), and cooled to a temperature of about 16°C. A 60 ml addition funnel was charged with para-cymene (39.87 g), neohexene (6.25 g), and diisobutylene-2 (8.34 g). The mixture was added from the addition funnel over 1 one hour and stirred for an additional 0.5 hours while maintaining the temperature at about 16°C. The reaction was then quenched with deionized water (15 ml), the layers separated, and the organic phase washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (45.17 g) containing 26.28 weight % HMT (75.13% molar yield of HMT based on the amount of neohexene charged).

### Example 3

A 100 ml, four-necked, round bottom flask was charged with methyltrioctylammonium chloride (1.063 g), cyclohexane (7.15 g), and anhydrous aluminum chloride (1.007 g) and cooled to about 16°C. Addition of para-cymene (39.87 g), neohexene (6.25 g) and diisobutylene-2 (8.34 g) was carried out from a 60 ml addition funnel over a period of about one hour while maintaining a flask temperature of about 16°C. The reaction was stirred an additional 30 minutes, then quenched with deionized water (15 ml). The organic phase was worked-up as in Example 1 to yield a crude product (43.07 g) containing 29.98 weight % HMT (81.72 molar yield of HMT based on the amount of neohexene charged).

### Example 4

A 100 ml, four-necked, round bottom flask was charged with Adogen-464™ (1.010 g), methylene chloride (12.16 g) and anhydrous aluminum chloride (1.005 g) and cooled to about 10°C. A 60 ml addition funnel was charged with para-cymene (39.87 g), diisobutylene-2 (8.34 g) and neohexene (6.25 g) and connected to the flask. The funnel mixture was then added to the flask over a period of about an hour, while maintaining a temperature of about 10-20°C. The reaction was quenched with deionized water (15 ml), and the organic phase separated and washed with, in order, with 5% HCl, 10% Na₂CO₃, and 50% brine solution. The aqueous layers were individually extracted with ethyl ether, and the ether layers combined with the organic phase. The organics were then dried over K₂CO₃, filtered, and evaporated to yield a crude product (44.41 g) containing 28.65 weight % HMT (80.53% molar yield of HMT based on the amount of neohexene charged).

### Example 5

A 100 ml, four-necked, round bottom flask was charged with methylene chloride (12.16 g) and anhydrous aluminum chloride (1.005 g) and cooled to about 16°C. A 60 ml addition funnel was charged with para-cymene (39.87 g), neohexene (6.25 g) and 2,4,6-trimethyl-3-heptene (10.22 g) and connected to the flask. The funnel mixture was added to the flask over a period of about one hour and the flask mixture stirred 0.5 hours following addition, while maintaining the temperature at about of 16°C. The reaction was then quenched with deionized water (15 ml), and the organic phase washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (40.10 g) containing 29.66 weight % HMT (75.28% molar yield of HMT based on the amount of neohexene charged).

### Example 6

A 100 ml, four-necked, round bottom flask was charged with methylene chloride (12.16 g) and anhydrous aluminum chloride (1.005 g) and cooled to about 16°C. A 60 ml addition funnel was charged with para-cymene (39.87 g), neohexene (6.25 g) and 2,4-dimethyl-2-pentene (7.30 g) and connected to the flask. The funnel mixture was added to the flask over a period of about one hour and the flask mixture stirred for about 0.5 hours following addition, while maintaining the temperature at about 16°C. The reaction was then quenched with deionized water (15 ml), and the organic phase separated and washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (42.56 g) containing 24.98 weight % HMT (67.29% molar yield of HMT based on the amount of neohexene charged).

### Example 7

A 50 ml, three-necked, round bottom flask was charged with methylene chloride (6.08 g) and anhydrous aluminum chloride (0.506 g) and cooled to about 16°C. A 60 ml addition funnel was charged with para-cymene (19.94 g), neohexene (3.13 g) and 3,4,4-trimethyl-2-pentene (4.17 g) and connected to the flask. The funnel mixture was added over a period of about 45 minutes and the flask mixture stirred an additional period of about 45 minutes. The reaction was then quenched with deionized water (10 ml), and the organic phase separated and washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (23.28 g) containing 19.45 weight % HMT (57.22% molar yield of HMT based on the amount of neohexene charged).

### Example 8

A 100 ml, four-necked, round bottom flask was charged with methylene chloride (12.16 g) and anhydrous aluminum chloride (1.007 g), cooled to about 15°C. A 60 ml addition funnel was charged with para-cymene (39.87 g), neohexene (6.25 g) and 2,3,4-trimethyl-2-pentene (8.34 g), and connected to the flask. While maintaining the temperature at about 15°C, the funnel mixture was added over a period of about one hour, and the flask mixture stirred for an additional period of about 30 minutes. The reaction was then quenched with deionized water (15 ml), and the organic phase separated and washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (40.54 g) containing 27.82 weight % HMT (71.38% molar yield of HMT based on the amount of neohexene charged).

### Example 9

A 100 ml, four-necked, round bottom flask was charged with methylene chloride (8.75 g) and anhydrous aluminum chloride (1.005 g) and cooled to about 15°C. A mixture of para-cymene (27.73 g), diisobutylene-2 (11.60 g), and neohexene (8.72 g) was charged into the flask using a syringe pump over a period of about 1.5 hours, as the flask ingredients were being stirred. The flask mixture was stirred an additional 0.5 hours, then quenched with deionized water (15 ml), and the organic phase separated and washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (33.85 g) containing 53.40 weight % HMT (82.00% molar yield of HMT based on the amount of neohexene charged).

### Example 10

A 100 ml, four-necked, round bottom flask was charged with methylene chloride (8.75 g) and anhydrous aluminum chloride (0.705 g) and cooled to about 15°C. A mixture of para-cymene and neohexene was charged into a 50 ml syringe. Into a second 50 ml syringe was charged para-cymene and diisobutylene-2. The syringe mixtures were added to the flask over a period of about one hour with the addition of the para-cymene/diisobutylene-2 mixture discontinued about six minutes before discontinuance of the para-cymene/neohexene mixture addition. The total amounts of the syringe ingredients added were 31.26 g of para-cymene, 8.77 g of neohexene and 9.46 g of diisobutylene-2. The reaction mixture was stirred for an additional 30 minutes, quenched with 10 ml deionized water (10 ml), and the organic phase separated and washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (35.80 g) containing 46.31 weight % HMT (74.78% molar yield of HMT, based on the amount of neohexene charged).

### Example 11

A 100 ml, four-necked, round bottom flask was charged with methylene chloride (12.16 g) and anhydrous aluminum chloride (1.003 g) and cooled to about 16°C. A 60 ml addition funnel was charged with para-cymene (39.87 g), diisobutylene-2 (8.34 g), and 3-methyl-1-hexene (7.29 g), and connected to the flask. The funnel mixture was added over a period of about one hour, and the flask mixture stirred for 0.5 hours following addition while maintaining the temperature at about 16°C. The reaction was then quenched with deionized water (15 ml), and the organic phase separated and washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (39.49 g) containing 23.82 weight % 1,1,4,6-tetramethyl-4-propyl-tetrahydronaphthalene (54.98% molar yield of the tetrahydronaphthalene product based on the amount of 3-methyl-1-hexene charged).

### Example 12

A 100 ml, four-necked, round bottom flask was charged with anhydrous aluminum chloride (1.006 g) and cyclohexene (7.15 g), and cooled to about 16°C. A 60 ml addition funnel was charged with para-cymene (39.87 g), 2,3-dimethyl-1-butene (6.25 g), and diisobutylene-2 (8.34 g) and connected to the flask. While maintaining a temperature of about 16°C, the funnel mixture was added to the flask over a period of about one hour and the flask mixture stirred an additional 30 minutes. The reaction was then quenched with deionized water (12 ml), and the organic phase separated and washed with, in order, 5% HCl, 10% Na₂CO₃, and 50% brine (solution). The aqueous layers were individually extracted with ethyl ether and the ether layers combined with the organic phase. The organics were then dried over K₂CO₃, filtered, and evaporated to yield a crude product (42.67 g) containing 24.51 weight % HMT (65.20% molar yield of HMT based on the amount of 2,3-dimethyl-1-butene charged).

### Example 13

A 100 ml, four-necked, round bottom flask was charged with Adogen-464™ (1.046 g), cyclohexane (7.15 g), and anhydrous aluminum chloride (1.000 g), and cooled to about 16°C. A 60 ml addition funnel was charged with para-cymene (39.87 g), 2,3-dimethyl-1-butene (6.25 g) and diisobutylene-2 (8.34 g). While maintaining a temperature of about 16°C, the funnel mixture was added to the flask over a period of about one hour, and the flask mixture stirred an additional 30 minutes. The reaction was then quenched with deionized water (12 ml), the layers separated, and the organic phase washed with, in order, 5% HCl, 10% Na₂CO₃ and 50% brine solution. The aqueous layers were individually extracted with ethyl ether, and the ether layers combined with the organic phase. The organics were then dried over K₂CO₃, filtered, and evaporated to yield a crude product (43.28 g) containing 23.85 weight % HMT (64.35% molar yield of HMT based on the amount of 2,3-dimethyl-1-butene charged).

### Example 14

A 100 ml four-necked, round bottom flask was charged with methyltrioctylammonium chloride (2.73 g), anhydrous aluminum chloride (1.803 g) and cyclohexane (19.10 g), and cooled to about 20°C. A mixture containing para-cymene (25.13 g), 2,3-dimethyl-1-butene (7.63 g), and diisobutylene-2 (11.53 g), was added, with rapid stirring, to the flask over a period of about 3 hours. The reaction was immediately quenched with deionized water (15 ml) and worked up as previously described in Example 11 to give a crude product (31.16 g) containing 41.66 weight % HMT (66.16% molar yield of HMT based on the amount of 2,3-dimethyl-1-butene charged).

### Example 15

A 100 ml, four-necked, round bottom flask was charged with methytrioctylammonium chloride (1.047 g), cyclohexane (7.15 g), and anhydrous aluminum chloride (1.001 g), and cooled to about 16°C. A mixture of para-cymene (39.87 g), 2,3-dimethyl-1-butene (6.25 g) and diisobutylene-2 (8.34 g) was added to the flask from a 60 ml addition funnel over a period of about one hour while maintaining a flask temperature of about 16°C. The mixture was stirred for 30 minutes following addition, and the reaction quenched with deionized water (15 ml). The organic phase was worked-up as in Example 11 to yield a crude product (42.79 g) containing 25.16 weight % HMT (67.12 molar yield of HMT based on the amount of 2,3-dimethyl-1-butene charged).

## Claims

1. A process for producing a polyalkyl tetrahydronaphthalene compound comprising contacting a partially substituted benzene compound, wherein said benzene compound is substituted with two or more substituents that do not substantially interfere with a Friedel-Crafts-type alkylation reaction said substituents including at least one secondary alkyl group having only one alpha-hydrogen, and wherein said benzene compound is unsubstituted in at least one position adjacent to said secondary alkyl group, with an olefinic compound of the Formula 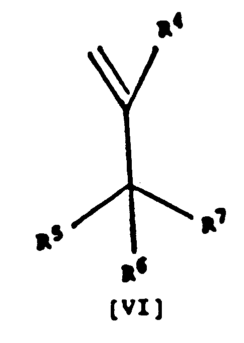 wherein R⁴, R⁵, R⁶ and R⁷ independently are substituents that do not substantially interfere with a Friedel-Crafts-type alkylation reaction provided that no more than one of R⁵, R⁶ and R⁷ are H, in the presence of
a further olefin reagent provided that said further olefin reagent has greater electron releasing properties than said olefinic compound of the formula VI, and
a Lewis acid,
characterised in that the further olefin reagent has the formula VII 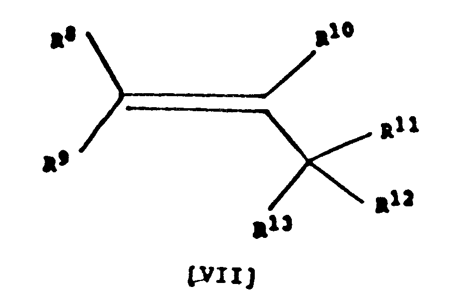 wherein
R⁸, R⁹, R¹⁰, R¹¹, R¹² and R¹³, independently, are substitutents that do not substantially interefere with a Freidel-Crafts-type alkylation reaction, provided that
(i) no more than one of R⁸, R⁹ and R¹⁰ are H, and
(ii) no more than one of R¹¹, R¹² and R¹³ are H.

2. A process of claim 1 in which the process is carried out in the substantial absence of elemental iodine.

3. A process of claim 1 or claim 2 carried out in the presence of a phase transfer agent.

4. A process according to any preceding claim for producing a polyalkyl tetrahydronaphthalene compound of the Formulas 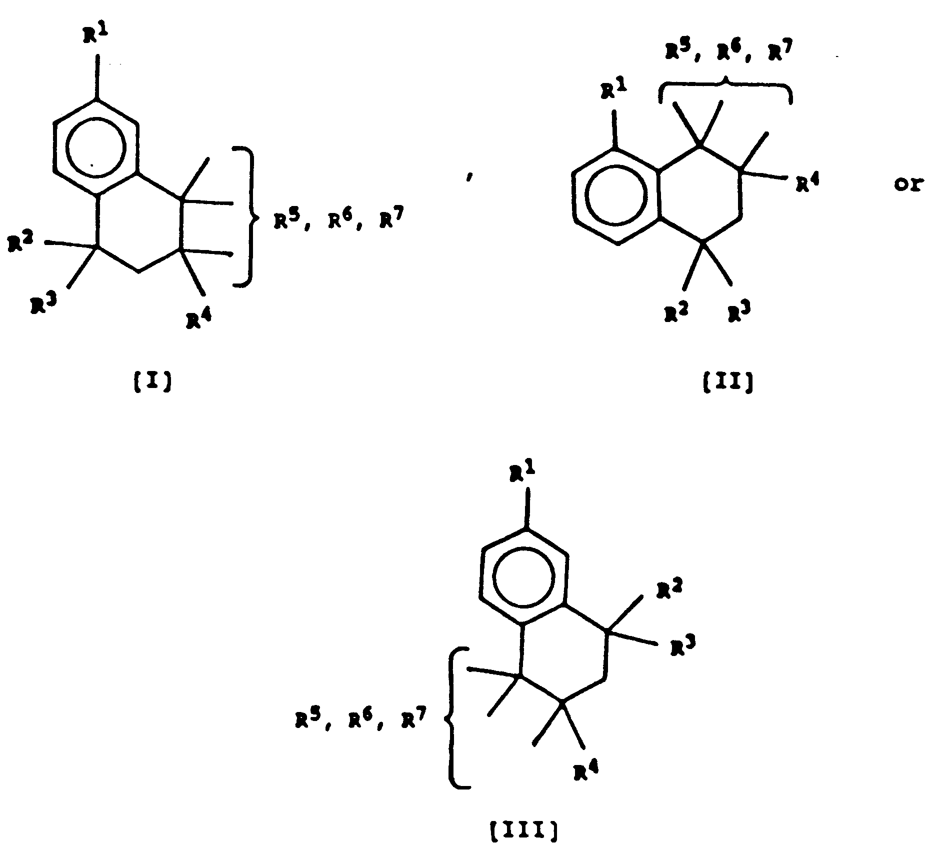 in which the partially substituted benzene compound is selected from 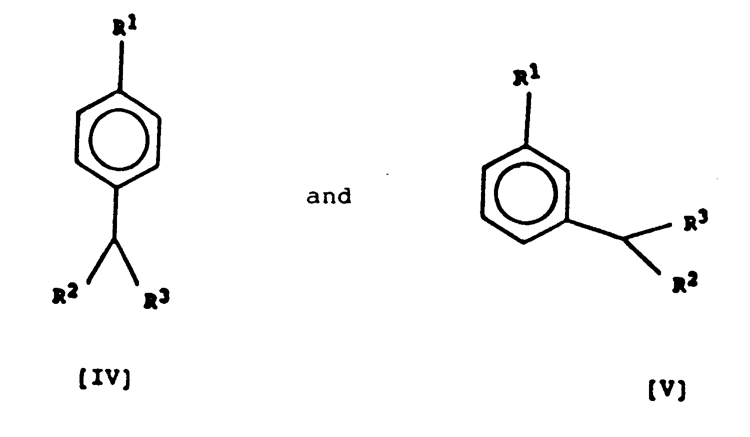 wherein
R¹, R² and R³, independently, are substituents that do not substantially interfere with a Friedel-Crafts-type alkylation reaction, provided that
(i) R¹, R² and R³ are each other than H.

5. A process of claim 4 wherein
R¹, R² and R³, independently, are a C₁-C₃₀ straight chain, branched or cyclical alkyl; and
R⁴, R⁵, R⁶, R⁷, R⁸, R⁹, R¹⁰, R¹¹, R¹² and R¹³, independently, are H, or a C₁-C₃₀ straight chain, branched or cyclical alkyl.

6. A process of claim 5 wherein said alkyl is a C₁-C₅ straight chain or branched alkyl.

7. A process of any of claims 4 to 6 wherein said polyalkyl tetrahydronaphthalene compound is of the Formula [I].

8. A process of claim 7 wherein
R¹, R², R³, R⁵, R⁶ and R⁷, independently, are a C₁-C₅ straight chain or branched alkyl; and
R⁴ is H.

9. A process of claim 8 wherein
R², R³, R⁵, R⁶ and R⁷ are each methyl and
R¹ is selected from methyl, ethyl, n-propyl and t-butyl.

10. A process of claim 7 wherein R¹, R², R³, R⁴, R⁵ and R⁶ are methyl and R⁷ is H.

11. A process of any of claims 5 to 10 wherein
R⁸, R⁹, R¹⁰, R¹¹, R¹² and R¹³, independently, are H or a C₁-C₅ straight chain or branched alkyl.

12. A process of claim 11 wherein
R⁸, R⁹, R¹¹, R¹² and R¹³ are each methyl; and
R¹⁰ is H.

13. A process of any of claims 5 to 12 wherein said Lewis acid is aluminum chloride.

14. A process of any of claims 5 to 13 further comprising a solvent.

15. A process of claim 3 wherein phase transfer agent is selected from the group consisting of ammonium, phosphonium and sulfonium salts.

16. A process of claim 15 wherein the ammonium salt is a quaternary ammonium halide.

17. A process according to any preceding claim in which the Lewis acid is present in an amount in the range 2 to 10% by weight - based on the total weight of the substituted benzene, the olefinic compound of the Formula VI and the olefin reagent of the Formula VII.

## Patentansprüche

1. Verfahren zur Herstellung einer Polyalkyltetrahydronaphthalin-Verbindung, welches umfaßt
das Inkontaktbringen einer partiell substituierten Benzolverbindung, worin die Benzolverbindung mit zwei oder mehr Substituenten, die eine Alkylierungsreaktion vom Friedel-Crafts-Typ nicht wesentlich stören, substituiert ist, welche Substituenten mindestens eine sekundäre Alkylgruppe mit nur einem Alpha-Wasserstoff einschließen, und worin die Benzolverbindung in mindestens einer zur sekundären Alkylgruppe benachbarten Position unsubstituiert ist,
mit einer olefinischen Verbindung der Formel 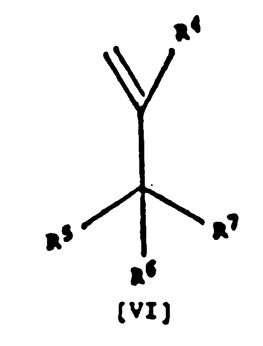 worin R⁴, R⁵, R⁶ und R⁷ unabhängig voneinander Substituenten sind, die eine Alkylierungsreaktion vom Friedel-Crafts-Typ nicht wesentlich stören, mit der Maßgabe, daß nicht mehr als eines von R⁵, R⁶ und R⁷ H ist,
in Gegenwart eines weiteren olefinischen Reagens,
mit der Maßgabe, daß das weitere olefinische Reagens größere Elektronen-freisetzende Eigenschaften besitzt als die olefinische Verbindung der Formel VI, und
einer Lewis-Säure,
dadurch gekennzeichnet, daß das weitere olefinische Reagens die Formel VII hat 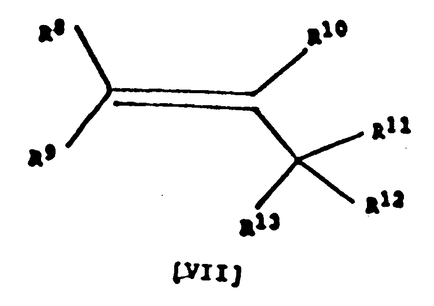 worin R⁸, R⁹, R¹⁰, R¹¹, R¹² und R¹³ unabhängig voneinander Substituenten sind, die eine Alkylierungsreaktion vom Friedel-Crafts-Typ nicht wesentlich stören,
mit der Maßgabe, daß
(i) nicht mehr als eines von R⁸, R⁹ und R¹⁰ H ist, und
(ii) nicht mehr als eines von R¹¹, R¹² und R¹³ H ist.

2. Verfahren nach Anspruch 1, worin das Verfahren in der im wesentlichen Abwesenheit von elementarem Iod durchgeführt wird.

3. Verfahren nach Anspruch 1 oder 2, welches in Anwesenheit eines Phasentransfer-Agens durchgeführt wird.

4. Verfahren nach irgendeinem der vorherigen Ansprüche, zur Herstellung einer Polyalkyltetrahydronaphthalin-Verbindung der Formeln 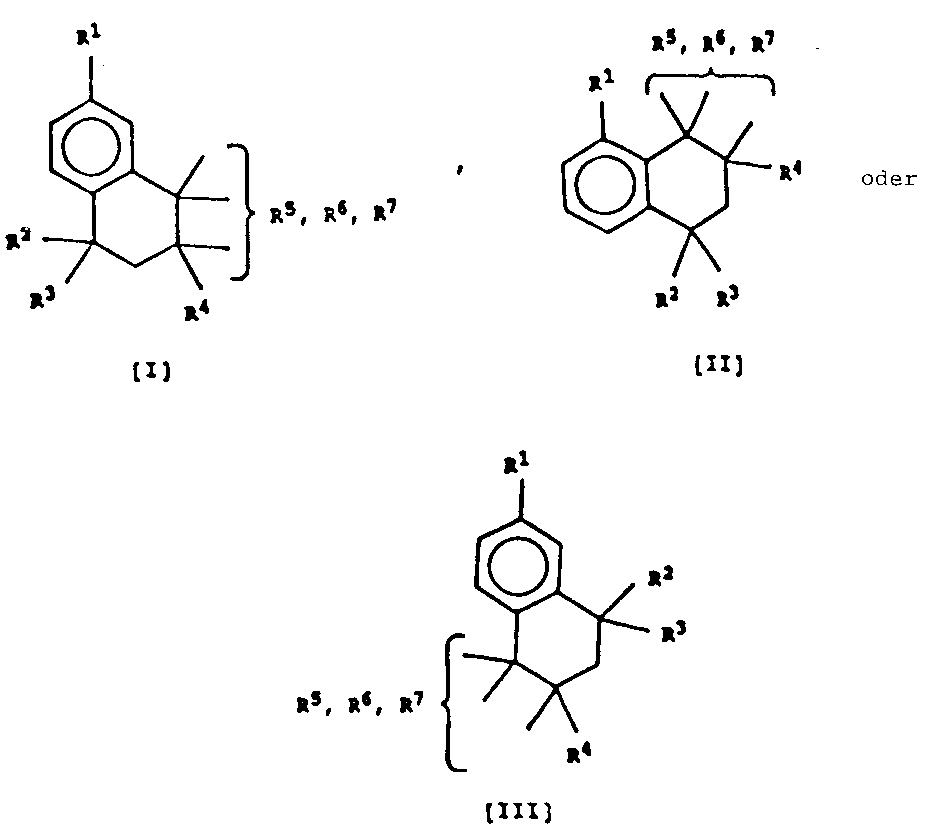 worin die partiell substituierte Benzolverbindung ausgewählt ist aus 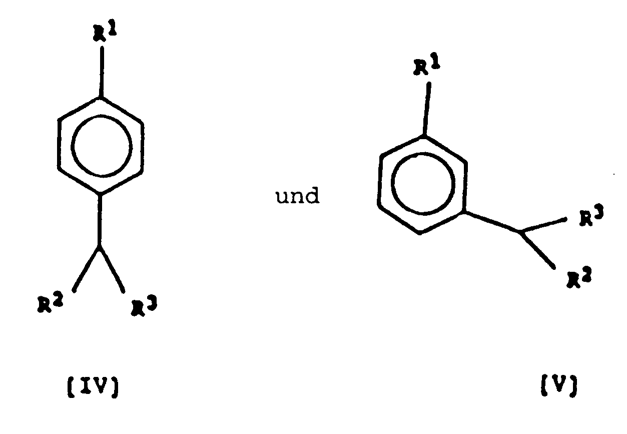 worin R¹, R² und R³ unabhängig voneinander Substituenten sind, die eine Alkylierungsreaktion vom Friedel-Crafts-Typ nicht wesentlich stören, mit der Maßgabe, daß
(i) R¹, R² und R³ jeweils nicht H sind.

5. Verfahren nach Anspruch 4, worin R¹, R² und R³ unabhängig voneinander ein geradkettiges, verzweigtes oder cyclisches C₁-C₃₀-Alkyl sind; und
R⁴, R⁵, R⁶, R⁷, R⁸, R⁹, R¹⁰, R¹¹, R¹² und R¹³ unabhängig voneinander H oder ein geradkettiges, verzweigtes oder cyclisches C₁-C₃₀-Alkyl sind.

6. Verfahren nach Anspruch 5, worin das Alkyl ein geradkettiges oder verzweigtes C₁-C₅-Alkyl ist.

7. Verfahren nach irgendeinem der Ansprüche 4 bis 6, worin die Polyalkyltetrahydronaphthalin-Verbindung eine der Formel (I) ist.

8. Verfahren nach Anspruch 7, worin R¹, R², R³, R⁵, R⁶ und R⁷ unabhängig voneinander ein geradkettiges oder verzweigtes C₁-C₅-Alkyl sind; und R⁴ H ist.

9. Verfahren nach Anspruch 8, worin R², R³, R⁵, R⁶ und R⁷ jeweils Methyl sind und R¹ ausgewählt ist aus Methyl, Ethyl, n-propyl und t-Butyl.

10. Verfahren nach Anspruch 7, worin R¹, R², R³, R⁴, R⁵ und R⁶ Methyl sind und R⁷ H ist.

11. Verfahren nach irgendeinem der Ansprüche 5 bis 10, worin R⁸, R⁹, R¹⁰, R¹¹, R¹² und R¹³ unabhängig voneinander H oder ein geradkettiges oder verzweigtes C₁-C₅-Alkyl sind.

12. Verfahren nach Anspruch 11, worin R⁸, R⁹, R¹¹, R¹² und R¹³ jeweils Methyl sind; und R¹⁰ H ist.

13. Verfahren nach irgendeinem der Ansprüche 5 bis 12, worin die Lewis-Säure Aluminiumchlorid ist.

14. Verfahren nach irgendeinem der Ansprüche 5 bis 13, welches weiterhin ein Lösungsmittel umfaßt.

15. Verfahren nach Anspruch 3, worin das Phasentransfer-Agens aus der Gruppe aus Ammonium-, Phosphonium- und Sulfoniumsalzen ausgewählt ist.

16. Verfahren nach Anspruch 15, worin das Ammoniumsalz ein quaternäres Ammoniumhalogenid ist.

17. Verfahren nach irgendeinem der vorhergehenden Ansprüche, worin die Lewis-Säure in einem Mengenbereich von 2 bis 10 Gew.-% - bezogen auf das Gesamtgewicht des substituierten Benzols, der olefinischen Verbindung der Formel VI und des olefinischen Reagens der Formel VII - anwesend ist.

## Revendications

1. Procédé pour produire un composé polyalkyltétrahydronaphtalène consistant à mettre en contact un composé benzénique partiellement substitué, dans lequel ledit composé benzénique est substitué par deux ou plusieurs substituants qui n'interfèrent pas sensiblement avec une réaction d'alkylation de type Friedel et Crafts, lesdits substituants incluant au moins un groupe alkyle secondaire n'ayant qu'un atome d'hydrogène en alpha, et dans lequel ledit composé benzénique est non substitué dans au moins une position adjacente audit groupe alkyle secondaire, avec un composé oléfinique de formule 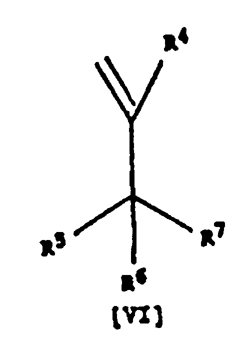 dans laquelle R⁴, R⁵, R⁶ et R⁷ représentent, indépendamment les uns des autres, des substituants qui n'interfèrent pas sensiblement avec une réaction d'alkylation de type Friedel et Crafts à condition que pas plus d'un de R⁵, R⁶ et R⁷ soit un atome H, en présence
- d'un réactif oléfinique supplémentaire à condition que ledit réactif oléfinique supplémentaire ait des propriétés de libération d'électrons plus importantes que ledit composé oléfinique de formule VI, et
- d'un acide de Lewis,
caractérisé en ce que le réactif oléfinique supplémentaire possède la formule VII 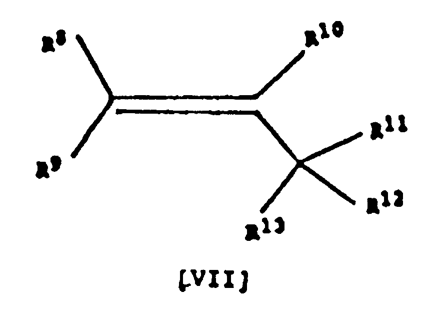 dans laquelle
R⁸, R⁹, R¹⁰, R¹¹, R¹² et R¹³, indépendamment les uns des autres, sont des substituants qui n'interfèrent pas sensiblement avec une réaction d'alkylation de type Friedel et Crafts, à condition que
(i) pas plus d'un de R⁸, R⁹ et R¹⁰ représente un atome H, et
(ii) pas plus d'un de R¹¹, R¹² et R¹³ représente un atome H.

2. Procédé selon la revendication 1, dans lequel le procédé est effectué en l'absence pratique d'iode élémentaire.

3. Procédé selon la revendication 1 ou 2, effectué en présence d'un agent de transfert de phase.

4. Procédé selon l'une quelconque des revendications précédentes, pour produire un composé polyalkyltétrahydronaphtalène des formules 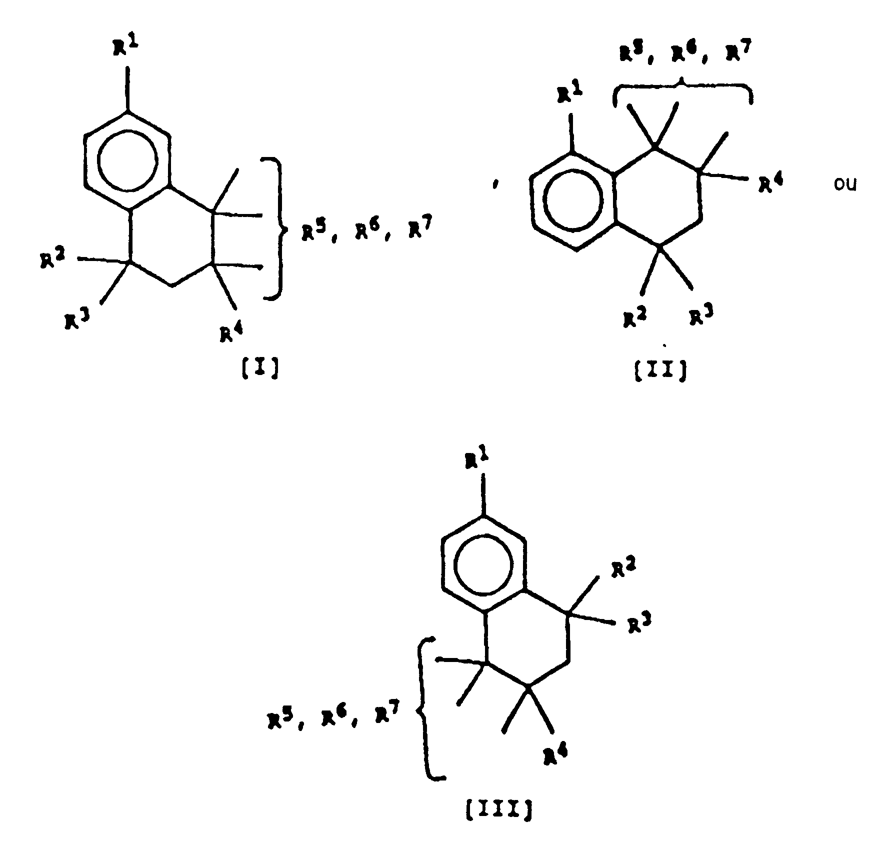 dans lesquelles le composé benzénique partiellement substitué est choisi parmi 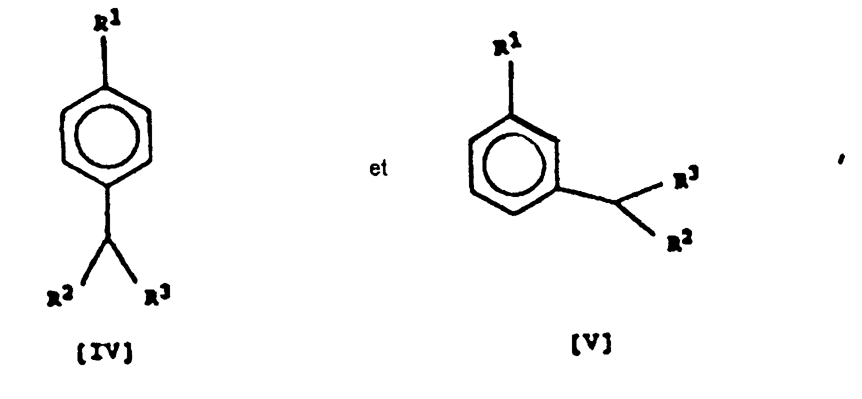 dans lesquelles
R¹, R² et R³, indépendamment les uns des autres, représentent des substituants qui n'interfèrent sensiblement pas avec une réaction d'alkylation de type Friedel et Crafts, à condition que
(i) R¹, R² et R³ soient chacun autre qu'un atome H.

5. Procédé selon la revendication 4, dans lequel
R¹, R² et R³, indépendamment les uns des autres, représentent un groupe alkyle cyclique, à chaîne droite ou ramifiée en C₁ à C₃₀; et
R⁴, R⁵, R⁶, R⁷, R⁸, R⁹, R¹⁰, R¹¹, R¹² et R¹³, indépendamment les uns des autres, représentent un atome H, ou un groupe alkyle cyclique, à chaîne droite ou ramifiée en C₁ à C₃₀.

6. Procédé selon la revendication 5, dans lequel ledit groupe alkyle est un groupe alkyle à chaîne droite ou ramifiée en C₁ à C₅.

7. Procédé selon l'une quelconque des revendications 4 à 6, dans lequel ledit composé polyalkyltétrahydronaphtalène est de formule [I].

8. Procédé selon la revendication 7, dans lequel
R¹, R², R³, R⁵, R⁶ et R⁷, indépendamment les uns des autres, sont un groupe alkyle à chaîne droite ou ramifiée en C₁ à C₅; et
R⁴ est un atome H.

9. Procédé selon la revendication 8, dans lequel
R², R³, R⁵, R⁶ et R⁷ sont chacun un groupe méthyle et
R¹ est choisi parmi les groupes méthyle, éthyle, n-propyle et t-butyle.

10. Procédé selon la revendication 7, dans lequel R¹, R², R³, R⁴, R⁵ et R⁶ sont un groupe méthyle et R⁷ est un atome H.

11. Procédé selon l'une quelconque des revendications 5 à 10, dans lequel R⁸, R⁹, R¹⁰, R¹¹, R¹² et R¹³, indépendamment les uns des autres, sont un atome H ou un groupe alkyle à chaîne droite ou ramifiée en C₁ à C₅.

12. Procédé selon la revendication 11, dans lequel
R⁸, R⁹, R¹¹, R¹² et R¹³ sont chacun un groupe méthyle; et
R¹⁰ est un atome H.

13. Procédé selon l'une quelconque des revendications 5 à 12, dans lequel ledit acide de Lewis est le chlorure d'aluminium.

14. Procédé selon l'une quelconque des revendications 5 à 13, comprenant de plus un solvant.

15. Procédé selon la revendication 3, dans lequel un agent de transfert de phase est choisi dans le groupe formé par les sels d'ammonium, de phosphonium et de sulfonium.

16. Procédé selon la revendication 15, dans lequel le sel d'ammonium est un halogénure d'ammonium quaternaire.

17. Procédé selon l'une quelconque des revendications précédentes, dans lequel l'acide de Lewis est présent en une quantité comprise entre 2 et 10% en poids par rapport au poids total du benzène substitué, du composé oléfinique de formule VI et du réactif oléfinique de formule VII.
